# EUROPÄISCHE PATENTSCHRIFT

(11) **EP 0 606 554 B1**
(45) Veröffentlichungstag und Bekanntmachung des Hinweises auf die Patenterteilung: **20.08.1997**
(21) Anmeldenummer: 93118001.2
(22) Anmeldetag: 05.11.1993
(51) Int. Cl.: A61N 1/05, A61N 1/375

(54) **Vorrichtung zum fixieren eines Elektrodenkabels an einem Gerät**
Device for securing an electrode lead in an apparatus
Dispositif pour fixer d'un fil d'électrode dans un appareil

(30) Priorität: 12.01.1993 SE 9300059
(43) Veröffentlichungstag der Anmeldung: 20.07.1994
(73) Patentinhaber: Pacesetter AB, 171 95 Solna (SE)
(72) Erfinder: Fröberg, Paul, S-161 70 Bromma (SE); Johansson, Göran, S-191 77 Sollentuna (SE)
(74) Vertreter: Lettström, Richard Wilhelm

(56) Entgegenhaltungen:
- DE-A- 2 347 720
- US-A- 4 784 141
- US-A- 4 934 367

## Beschreibung

Die Erfindung bezieht sich auf eine Vorrichtung zum Fixieren eines Elektrodenkabels an einem Gerät zum Abgeben von elektrischen Impulsen, insbesondere einem Herzschrittmacher oder einem Defibrillator, wobei das Gerät mit einem Anschlussteil versehen ist, in dem die Fixiervorrichtung für das proximale Ende des Elektrodenkabels angebracht ist, wobei das proximale Ende des Elektrodenkabels mit einem zentrisch angeordneten Kanal versehen ist.

In der DE-OS 2 518 571 ist eine Fixiervorrichtung der obengenannten Art beschrieben, die zum Anschliessen mindestens einer Herzschrittmacherelektrode an einem Herzschrittmacher vorgesehen ist. Die Fixiervorrichtung umfasst eine Schraube, die senkrecht zur Längsachse des Elektrodenkabels angeordnet ist und die von oben in das Anschlussteil geschraubt wird und das distale Ende des Elektrodenkabels punktfixiert. Um zu vermeiden, dass Körperflüssigkeiten in das Anschlussteil eindringen, ist die Öffnung für die Schraube mit einem Abdichtungsspund versehen. Durch die Schraube und den Abdichtungsspund wird ein verhältnismässig hohes Anschlussteil erhalten. Dies ist nicht wünschenswert, da danach gestrebt wird, dass der Herzschrittmacher die kleinstmögliche Dimension erhalten soll.

Durch die DE-OS 2 914 034 ist eine weitere Fixiervorrichtung für eine Herzschrittmacherelektrode bekannt, die in ihrem Aufbau der in der DE-OS 2 518 571 beschriebenen Fixiervorrichtung ähnelt mit dem Unterschied, dass die Fixierschraube und der Abdichtungsspund in dieser Ausführungsform derart angeordnet sind, dass sie das Elektrodenkabel von der einen Längsseite des Anschlussteils befestigen bzw. abdichten sollen, was mit sich bringt, dass das Anschlussteil verhältnismässig dick wird.

In der US-PS 4 934 367 ist ein Anschlussteil eines Herzschrittmachers beschrieben. Das Anschlussteil ist mit einem halsförmigen Teil versehen, das teilweise das proximale Ende des Elektrodenkabels umschliesst, wenn das Kabel im Anschlussteil appliziert ist. Als Fixiermittel für das Ende des Elektrodenkabels dient ein Suturgarn, das der implantierende Arzt um das halsförmige Teil bindet, was umständlich sein kann. Ausserdem ist eine solche Fixierung nicht ganz sicher.

DE-A-2 347 720 Figur 2 zeigt eine Vorrichtung zum Fixieren 27, 45 eines Elektrodenkabels 11 mit einem zentrisch angeordneten Kanal 22.

Der Erfindung liegt die Aufgabe zugrunde, eine Fixiervorrichtung der eingangs genannten Art zu schaffen, bei dem die Grösse des Anschlussteils des Geräts reduziert werden kann, gleichzeitig damit, dass eine sehr gute Fixierung des Elektrodenkabels erhalten wird.

Diese Aufgabe ist erfindungsgemäss dadurch gelöst, dass die Fixiervorrichtung ein langgestrecktes Organ umfasst, auf das das proximale Ende des Elektrodenkabels aufschiebbar ist sowie Mittel zur radialen Expansion wenigstens eines Teils des langgestreckten Organs, so dass bei einem aufgeschobenen Kabelende das Organ gegen die umgebende Kanalwand druckfixiert werden kann. Indem das langgestreckte Organ in den Kanal des Elektrodenkabels eingeschoben und im Kanal fixiert wird, kann die Fixiervorrichtung derart klein gemacht werden, dass deren Aussendurchmesser etwa dem Aussendurchmesser des proximalen Endes des Elektrodenkabels entspricht. Auf diese Weise kann die Grösse des Anschlussteils erheblich reduziert werden.

Im Hinblick auf eine günstige konstruktive Ausgestaltung der Erfindung empfiehlt es sich, dass das langgestreckte Organ rohrförmig ausgebildet ist und mit mindestens einem entlang der Längsachse des Organs verlaufenden Schlitz versehen ist, der sich über einen Teil der Länge des Rohres erstreckt, wobei die Expansionsmittel wenigstens entlang eines Teiles der Länge des Schlitzes in dem Rohr verschiebbar angebracht sind.

Gemäß einem Ausführungsbeispiel bestehen die Expansionsmittel aus einem Element, dessen Aussendurchmesser grösser als der Innendurchmesser derjenigen Strecke des Rohres ist, die mit dem Schlitz versehen ist. Die Expansionsmittel sollten eine derartige Form aufweisen, dass sie in einfacher Weise in das Rohr einführbar sind. Beispiele dafür sind Konus- oder Kugelformen.

In einer vorteilhaften Ausbildung der Erfindung wird vorgeschlagen, dass der Innendurchmesser des Rohres ausserhalb der Schlitzstrecke grösser als der oder gleich dem Aussendurchmesser der Expansionsmittel ist. Hierdurch wird ereicht, daß sich die Expansionsmittel im Rohr befinden können, ohne in eine expandierte Lage gebracht zu sein, was das Aufschieben des Endes des Elektrodenkabels vereinfachen kann.

Eine vorteilhafte Weiterbildung der Erfindung ergibt sich daraus, dass das langgestreckte Organ mit mindestens zwei Schlitzen versehen ist, die gleich verteilt über die Mantelfläche des Organs von dem Ende des Organs, auf das das Elektrodenkabel aufschiebbar ist, verlaufen.

Gemäß einem Ausführungsbeispiel kann das Organ aus einem federnden Material bestehen. Die Wahl des Materials ist von grosser Bedeutung, da das Organ dadurch seine ursprüngliche Dimension wieder einnehmen kann, wenn die Expansionsmittel in eine Lage verschoben werden, in der das Rohr nicht beeinflusst wird. Auf diese Weise kann sich das Ende des Elektrodenkabels vom Organ lösen.

In einer weiteren vorteilhaften Ausbildung der Erfindung wird vorgeschlagen, dass die Fixiervorrichtung auch eine erste Halterung, die am Anschlussteil des Gerätes fest angebracht ist, umfasst und dass das eine Ende des Organs an der Halterung derart befestigt ist, dass die Längsachse der Halterung mit der Längsachse des Organs achsfluchtend liegt. Durch diesen Aufbau ist die Fixieranordnung an die langgestreckten Konfiguration des Herzschrittmachers bzw. des Anschlussteils angepasst.

In einer vorteilhaften Weiterbildung der Erfindung wird vorgeschlagen, dass die Fixiervorrichtung auch eine zweite Halterung umfasst, die gegenüber der ersten Halterung verschiebbar angeordnet und mit den Expansionsmitteln verbunden ist. Hierdurch wird erreicht, dass die Expansionsmittel in einer definierten Weise gesteuert werden können.

Eine besonders günstige Ausgestaltung wird erhalten, indem die zweite Halterung über ein in der Längsrichtung steifes Verbindungselement mit den Expansionsmitteln verbunden ist. Durch das steife Verbindungselement können die Expansionsmittel in beiden Richtungen in dem Rohr verschoben werden.

Eine weitere günstige Ausgestaltung ergibt sich, wenn die beiden Halterungen mittels eines Gewindes miteinander verbunden sind, wobei die zweite Halterung gegenüber der ersten Halterung durch Drehen der ersten Halterung verschiebbar ist, wobei beim Drehen der Halterung in die eine Richtung das Organ expandiert und beim Drehen der Halterung in die andere Richtung das Organ seine ursprüngliche Form einnimmt.

Die Erfindung ist nachfolgend anhand von mehreren in den Zeichnungen dargestellten Ausführungsbeispielen näher erläutert. Es zeigen:
- FIG 1 und 2: perspektivische Ansichteneiner Fixiervorrichtung nach der Erfindung in einer expandierten und in einer nicht expandierten Lage.
- FIG 3: eine perspektivische Ansicht einer Fixiervorrichtung nach der Erfindung in einer weiteren Ausführungsform als der, die in der FIG 1 gezeigt ist und in einer nicht expandierten Lage.
- FIG 4: einen Querschnitt durch die Fixiervorrichtung nach der Schnittlinie IV - IV in der FIG 3 und
- FIG 5: eine perspektivisiche Ansicht der Fixiervorrichtung nach der FIG 3 in einer expandierten Lage.

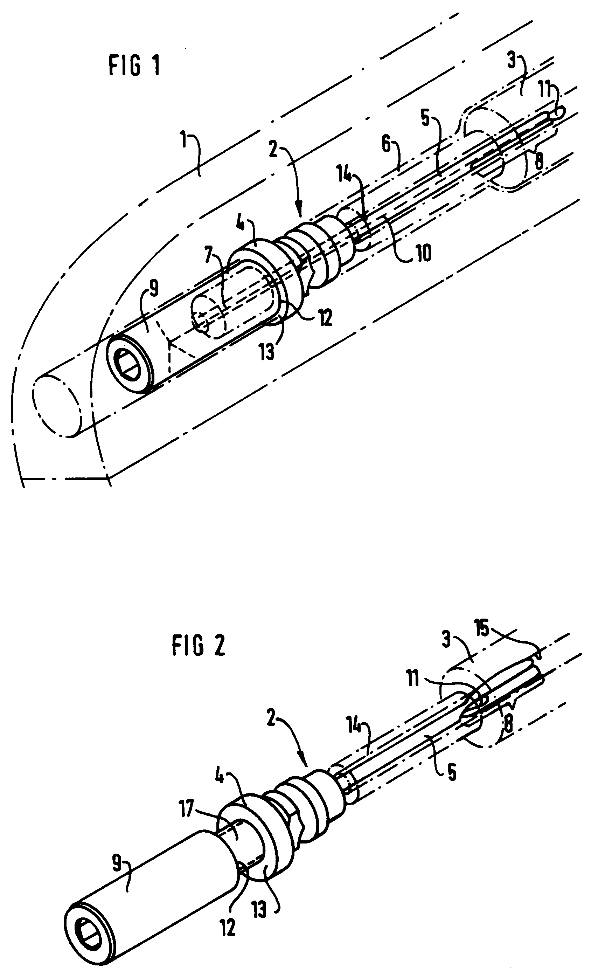

In der FIG 1 sind mit Hilfe von punktgestrichelten Linien die Konturen eines Anschlussteils 1 eines Herzschrittmachers gezeigt. In dem Anschlussteil 1 ist eine Fixiervorrichtung 2 zum Fixieren des proximalen Endes eines Elektrodenkabels 3 angebracht. Die Fixiervorrichtung 2 umfasst eine erste Halterung 4 mit einem achsial durchgehenden Kanal 7, wobei die Halterung 4 im Anschlussteil 1 fest angebracht ist. An der Halterung 4 ist ein langgestrecktes rohrförmiges Organ 5 derart befestigt, dass die Längsachse der Halterung 4 mit der Längsachse des rohrförmigen Organs bzw. des Rohres 5 achsfluchtend liegt. Das Rohr 5 ist seinerseits in einer Öffnung 6 am Anschlussteil 1, in die das Kabelende 3 einführbar ist, zentriert angebracht. Das Rohr 5 ist in diesem Ausführungsbeispiel mit zwei Schlitzen versehen, wobei lediglich der Schlitz 8 in dieser FIG 1 dargestellt ist. Die Schlitze 8 verlaufen von dem freien Ende des Rohres 5 über einen Teil von dessen Länge. Die Fixiervorrichtung umfasst auch Expansionsmittel 11, deren Aussendurchmesser grösser als der Innendurchmesser des Rohres 5 sind und die in das Rohr 5 derart einführbar sind, dass sie den Teil des Rohres, der mit den Schlitzen 8 versehen ist, radial expandieren. Die Fixiervorrichtung 2 umfasst auch eine zweite Halterung 9, die mittels eines Gewindes 17 gegenüber der ersten Halterung 4 verschiebbar angeordnet ist und über ein Verbindungselement 10 mit den Expansionsmitteln 11 verbunden ist. Das Verbindungselement 10, das in der Längsrichtung steif ist, verläuft durch den Kanal 7 der ersten Halterung 4 und durch das Rohr 5. Wenn die zweite Halterung 9 mit der ersten Halterung 4 derart zusammengeschraubt ist, dass die Stirnseite 12 der zweiten Halterung 9 gegen die Stirnseite 13 der ersten Halterung 4 liegt, kommen die Expansionsmittel 11 ausserhalb des Rohres 5 zu liegen. In dieser Lage kann das Kabelende 3, das mit einem zentrisch angeordneten Kanal 14 versehen ist, der zum Einschieben eines Mandrins vorgesehen ist, auf das Rohr 5 geschoben werden. Danach kann die zweite Halterung 9 mittels eines hier nicht gezeigten Werkzeuges derart gedreht werden, dass diese Halterung 9 gegenüber der ersten Halterung 4 verschoben wird, wodurch die Expansionsmittel 11 in das Rohr 5 zwischen den Schlitzen 8 hinein verschoben werden, so dass das Rohr 5 im Bereich der Schlitze 8 in radialer Richtung expandieren, wobei die Mantelflächen des Rohres 5 zwischen den Schlitzen 8 gegen die umgebende Kanalwand 15, wie es in der FIG 2 gezeigt wird, druckfixiert werden. Durch Drehen der zweiten Halterung 9, so dass die Expansionsmittel 11 aus dem Rohr 5 hinausverschoben werden, nimmt das Rohr 5 wieder seine ursprüngliche Lage ein. Dies erfolgt, wenn das Rohr aus einem federnden Material besteht. Hierdurch kann bei einem Wechseln eines Herzschrittmachers oder eines Elektrodenkabels das Kabelende 3 von der Fixiervorrichtung 2 gelöst werden. Die Expansionsmittel 11 sollten eine derartige Form aufweisen, dass sie in den geschlitzten Abschnitt des Rohres 5 eingeführt werden können. In diesem Fall ist ein Expansionsmittel mit einer Kugel- oder Konusform geeignet.

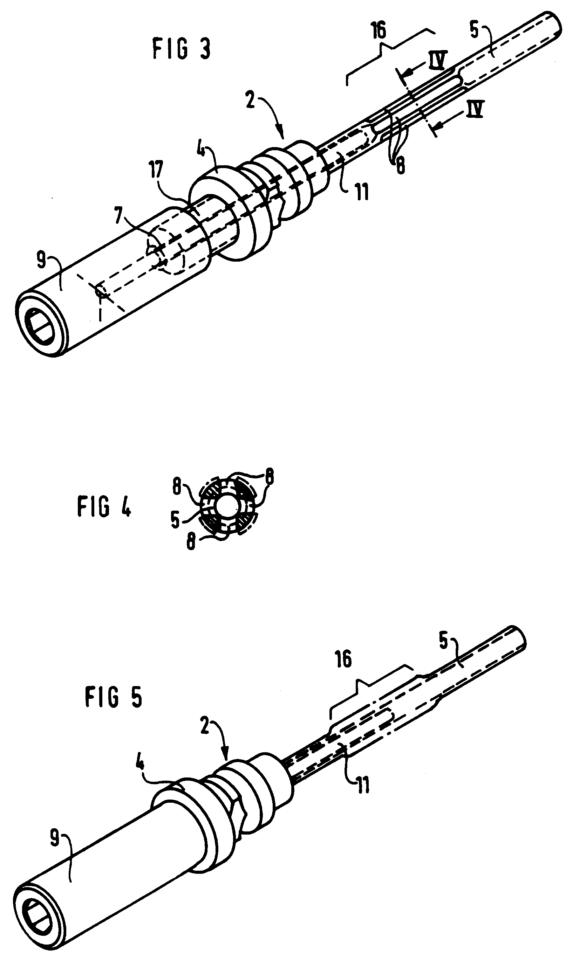

In der FIG 3 sind die Schlitze 8 am Rohr 5 der Fixiervorrichtung etwa in der Mitte angebracht, gleichzeigit damit, dass der Innendurchmesser ausserhalb der Schlitzstrecke 16 etwas grösser als der Aussendurchmesser der Expansionsmittel 11 ist. Mit diesem Ausführungsbeispiel wird gezeigt, dass die Expansionsmittel 11 im Rohr 5 angeordnet sein können, auch wenn das Rohr 5 in eine nicht expandierte Lage gebracht ist, was einen insgesamt kompakten Eindruck der Fixiervorrichtung ergibt. Mit den FIG 3 und 5 soll auch gezeigt werden, dass durch Drehen der zweiten Halterung 9, so dass dessen Stirnseite 12 gegen die Stirnseite 13 der ersten Halterung 4 anliegt, die Expansionsmittel in dem Rohr 5 entsprechend weit verschoben werden, so dass sie wenigstens teilweise im Bereich der Schlitzstrecke 16 liegen, wodurch wenigstens dieser Teil des Rohres 5 expandiert. Hierdurch werden die Mantelflächen zwischen den Schlitzen 8 bei einem aufgeschobenen Kabelende gegen die umgebende Kanalwand 15 druckfixiert.

In der FIG 4, die einen Querschnitt durch das Rohr 5 in FIG 3 darstellt, ist das Rohr 5 teils in einer nicht expandierten Lage und teils in einer expandierten Lage gezeigt. Die expandierte Lage wird mit Hilfe der punktgestrichelten Konturen veranschaulicht, die das Rohrgut zwischen den Schlitzen 8 darstellen sollen.

In dem Ausführungsbeispiel nach den FIG 3 bis 5 können die Gewindekämme an der ersten Halterung 4 abgerundet sein. Durch diese Gewindeform kann die zweite Halterung 9 gegenüber der ersten Halterung 4 verschoben werden, wobei ein sehr schneller Verschluss des Kabelendes erzielt wird. Wenn das Kabelende vom Rohr 5 gelöst werden soll, wird die zweite Halterung 9 in einer in der FIG 3 gezeigten Lage zurückgeschraubt, wobei das Rohr 5 seine ursprüngliche Lage einnimmt.

### Bezugszeichenliste

- 1: Anschlussteil
- 2: Fixiervorrichtung
- 3: Elektrodenkabel, Kabelende
- 4: erste Halterung
- 5: Rohr, Organ
- 6: Öffnung
- 7: Kanal
- 8: Schlitz
- 9: zweite Halterung
- 10: Verbindungselement
- 11: Expansionsmittel
- 12,13: Stirnseite
- 14: Kanal im Elektrodenkabel
- 15: Kanalwand
- 16: Schlitzstrecke
- 17: Gewinde

## Patentansprüche

1. Vorrichtung zum Fixieren eines Elektrodenkabels (3) an einem Gerät zum Abgeben von elektrischen Impulsen, insbesondere einem Herzschrittmacher oder einem Defibrillator, wobei das Gerät mit einem Anschlussteil (1) versehen ist, in dem die Fixiervorrichtung für das proximale Ende des Elektrodenkabels (3) angebracht ist; wobei das proximale Ende des Elektrodenkabels mit einem zentrisch angeordneten Kanal (14) versehen ist, dadurch **gekennzeichnet,** dass die Fixiervorrichtung (2) ein langgestrecktes Organ (5) umfasst, auf das das proximale Ende des Elektrodenkabels (3) aufschiebbar ist sowie Mittel (11) zur radialen Expansion wenigstens eines Teils des langgestreckten Organs (5), so dass bei einem aufgeschobenen Kabelende (3) das Organ (5) gegen die umgebende Kanalwand (15) druckfixiert werden kann.

2. Vorrichtung nach Anspruch 1, dadurch **gekennzeichnet,** dass das langgestreckte organ (5) rohrförmig ausgebildet ist und mit mindestens einem entlang der Längsachse des organs (5) verlaufenden Schlitz (8) versehen ist, der sich über einen Teil der Länge des Rohres (5) erstreckt, wobei die Expansionsmittel (11) wenigstens entlang eines Teiles der Länge des Schlitzes (8) in dem Rohr (5) verschiebbar angebracht sind.

3. Vorrichtung nach Anspruch 2, dadurch **gekennzeichnet,** dass die Expansionsmittel (11) aus einem Element besteht, dessen Aussendurchmesser grösser als der Innendurchmesser derjenigen Strecke des Rohres (5) ist, die mit dem Schlitz (8) versehen ist.

4. Vorrichtung nach Anspruch 2 oder 3, dadurch **gekennzeichnet,** dass der innendurchmesser des Rohres (5) ausserhalb der Schlitzstrecke (16) grösser als der oder gleich dem Aussendurchmesser der Expansionsmittel (11) ist.

5. Vorrichtung nach einem der Ansprüche 1 bis 4, dadurch **gekennzeichnet,** dass das langgestreckte organ (5) mit mindestens zwei Schlitzen (8) versehen ist, die gleich verteilt über die Mantelfläche des Organs (5) von dem Ende des Organs (5), auf das das Elektrodenkabel (3) aufschiebbar ist, verlaufen.

6. Vorrichtung nach einem der Ansprüche 1 bis 5, dadurch **gekennzeichnet,** dass das Organ (5) aus einem federnden Material besteht.

7. Vorrichtung nach einem der Ansprüche 1 bis 6, dadurch **gekennzeichnet,** dass die Fixiervorrichtung (2) auch eine erste Halterung (4), die am Anschlussteil (1) des Gerätes fest angebracht ist, umfasst und dass das eine Ende des Organs (5) an der Halterung derart befestigt ist, dass die Längsachse der Halterung (4) mit der Längsachse des Organs (5) achsfluchtend liegt.

8. Vorrichtung nach Anspruch 7, dadurch **gekennzeichnet,** dass die Fixiervorrichtung (2) auch eine zweite Halterung (9) umfasst, die gegenüber der ersten Halterung (4) verschiebbar angeordnet und mit den Expansionsmitteln (11) verbunden ist.

9. Vorrichtung nach Anspruch 8, dadurch **gekennzeichnet**, dass die zweite Halterung (9) über ein in der Längsrichtung steifes Verbindungselement (10) mit den Expansionsmitteln (11) verbunden ist.

10. Vorrichtung nach Anspruche 8 oder 9, dadurch **gekennzeichnet,** dass die beiden Halterungen (4, 9) mittels eines Gewindes (17) miteinander verbunden sind, wobei die zweite Halterung (9) gegenüber der ersten Halterung (4) durch Drehen der ersten Halterung (4) verschiebbar ist, wobei beim Drehen der Halterung (9) in die eine Richtung das Organ (5) expandiert und beim Drehen der Halterung (9) in die andere Richtung das Organ (5) seine ursprüngliche Form einnimmt.

## Claims

1. Device for fixing an electrode cable (3) to an apparatus for emitting electrical pulses, in particular to a heart pacemaker or a defibrillator, with the apparatus being provided with a connecting piece (1) in which the fixing device for the proximal end of the electrode cable (3) is mounted; with the proximal end of the electrode cable being provided with a centrally-arranged channel (14), characterised in that the fixing device (2) comprises an elongated element (5) onto which can be pushed the proximal end of the electrode cable (3), as well as means (11) for radial expansion of at least one portion of the elongated element (5), so that when the cable end (3) is pushed on, the element (5) can be pressure-fixed against the surrounding channel wall (15).

2. Device according to claim 1, characterised in that the elongated element (5) is constructed in a manner such that it is tubular and is provided with at least one slit (8) which extends along the longitudinal axis of the element (5) and extends over a portion of the length of the tube (5), with the expansion means (11) being mounted in a manner such that they can be shifted in the tube (5) at least along a portion of the length of the slit (8).

3. Device according to claim 2, characterised in that the expansion means (11) consist of an element, the outside diameter of which is larger than the inside diameter of the section of the tube (5) that is provided with the slit (8).

4. Device according to claim 2 or 3, characterised in that the inside diameter of the tube (5) beyond the slit section (16) is larger than or equal to the outside diameter of the expansion means (11).

5. Device according to one of the claims 1 to 4, characterised in that the elongated element (5) is provided with at least two slits (8), which extend in an evenly-distributed manner over the lateral surface of the element (5) from the end of the element (5) onto which the electrode cable (3) can be pushed.

6. Device according to one of the claims 1 to 5, characterised in that the element (5) consists of an elastic material.

7. Device according to one of the claims 1 to 6, characterised in that the fixing device (2) also comprises a first holding device (4), which is securely mounted on the connecting piece (1) of the apparatus, and in that the one end of the element (5) is secured to the holding device in such a way that the longitudinal axis of the holding device (4) lies in a manner such that it is axially aligned with the longitudinal axis of the element (5).

8. Device according to claim 7, characterised in that the fixing device (2) also comprises a second holding device (9), which is arranged in a manner such that it can be shifted with respect to the first holding device (4) and is connected to the expansion means (11).

9. Device according to claim 8, characterised in that the second holding device (9) is connected to the expansion means (11) by way of a joining element (10) which is rigid in the longitudinal direction.

10. Device according to claim 8 or 9, characterised in that the two holding devices (4, 9) are connected to each other by means of a thread (17), with the second holding device (9) being shiftable with respect to the first holding device (4) by rotating the first holding device (4), with the element (5) expanding when the holding device (9) is rotated in the one direction, and with the element (5) taking its original shape when the holding device (9) is rotated in the other direction.

## Revendications

1. Dispositif de fixation d'un câble (3) d'électrode à un appareil pour la fourniture d'impulsions électriques, notamment d'un stimulateur cardiaque ou d'un défibrillateur, l'appareil étant muni d'une pièce (1) de raccordement, dans laquelle le dispositif de fixation pour l'extrémité proximale du câble (3) d'électrode est mis en place, l'extrémité proximale du câble d'électrode étant munie d'un canal (14) disposé de manière centrale, **caractérisé en ce que** le dispositif (2) de fixation comprend un organe (5) oblong, sur lequel l'extrémité proximale du câble (3) d'électrode peut coulisser, ainsi que des moyens (11) pour l'extension radiale d'au moins une partie de l'organe (5) oblong, de sorte que l'organe (5) puisse être immobilisé par pression contre la paroi (15) du canal environnante lorsque l'extrémité (3) du câble est repoussée.

2. Dispositif suivant la revendication 1, **caractérisé en ce que** l'organe (5) oblong est de forme tubulaire et il est ménagé dans celui-ci au moins une fente (8) s'étendant le long de l'axe longitudinal de l'organe (5) et s'étendant sur une partie de la longueur du tuyau (5), les moyens (11) d'extension étant montés coulissant dans le tuyau (5) au moins le long d'une partie de la longueur de la fente (8).

3. Dispositif suivant la revendication 2, **caractérisé en ce que** les moyens (11) d'extension sont constitués d'un élément, dont le diamètre extérieur est plus grand que le diamètre intérieur de la section du tuyau (5), qui comporte la fente (8).

4. Dispositif suivant la revendication 2 ou 3, **caractérisé en ce que** le diamètre intérieur du tuyau (5) à l'extérieur du tronçon (16) fendu est supérieur ou égal au diamètre extérieur des moyens (11) d'extension.

5. Dispositif suivant l'une des revendications 1 à 4, **caractérisé en ce que** l'organe (5) oblong comporte au moins deux fentes (8), qui sont réparties de la même manière sur la surface latérale de l'organe (5) à partir de l'extrémité de l'organe (5), sur lequel le câble (3) d'électrode peut coulisser.

6. Dispositif suivant l'une des revendications 1 à 5, **caractérisé en ce que** l'organe (5) est en un matériau élastique.

7. Dispositif suivant l'une des revendications 1 à 6, **caractérisé en ce que** le dispositif (2) de fixation comprend également une première fixation (4), qui est mise en place à demeure sur la pièce (1) de raccordement de l'appareil et l'une des extrémités de l'organe (5) est fixée à la fixation de telle sorte que l'axe longitudinal de la fixation (4) soit aligné avec l'axe longitudinal de l'organe (5).

8. Dispositif suivant la revendication 7, **caractérisé en ce que** le dispositif (2) de fixation comprend également une seconde fixation (9), qui est montée coulissante par rapport à la première fixation (4) et qui est reliée aux moyens (11) d'extension.

9. Dispositif suivant la revendication 8, **caractérisé en ce que** la seconde fixation (9) est reliée aux moyens (11) d'extension par l'intermédiaire d'un élément (10) de liaison rigide dans le sens de la longueur.

10. Dispositif suivant la revendication 8 ou 9, **caractérisé en ce que** les deux fixations (4, 9) sont reliées entre elles au moyen d'un filetage (17), la seconde fixation (9) pouvant coulisser par rapport à la première fixation (4) par rotation de la première fixation (4), l'organe (5) étant étendu lorsque la fixation (9) tourne dans un sens et l'organe (5) prenant sa forme initiale lorsque la fixation (9) tourne dans l'autre sens.
